# EUROPEAN PATENT SPECIFICATION

(11) **EP 4 354 142 B1**
(45) Date of publication and mention of the grant of the patent: **17.12.2025**
(21) Application number: 22461622.7
(22) Date of filing: 15.10.2022
(51) Int. Cl.: G01N 33/574

(54) **RAMAN SPECTRA BASED METHOD OF IDENTIFYING THE SUBTYPE OF ACUTE LYMPHOBLASTIC LEUKEMIA WITH KMT2A GENE REARRANGEMENT**
RAMAN-SPEKTRA-BASIERTE METHODE ZUR IDENTIFIZIERUNG DES SUBTYPS DER AKUTEN LYMPHOBLASTISCHEN LEUKÄMIE MIT KMT2A-GENNEUANORDNUNG
PMÉTHODE BASÉE SUR LES SPECTRES RAMAN POUR IDENTIFIER LE SOUS-TYPE DE LEUCÉMIE LYMPHOBLASTIQUE AIGUË AVEC RÉARRANGEMENT DU GÈNE KMT2A

(43) Date of publication of application: 17.04.2024
(73) Proprietor: UNIWERSYTET JAGIELLONSKI, 31-007 Kraków (PL); Uniwersytet Medyczny w Lodzi, 90-419 Lodz (PL)
(72) Inventor: Majzner, Katarzyna, 30-349 Kraków (PL); Leszczenko, Patrycja, 32-091 Michalowice (PL); Nowakowska, Anna, 31-216 Kraków (PL); Baranska, Malgorzata, 32-087 Trojanowice (PL); Jakubowska, Justyna, 95-050 Konstantynów Lódzki (PL); Zabczynska, Marta, 91-803 Lódz (PL); Pastorczak, Agata, 90-229 Lódz (PL); Ostrowska, Kinga, 93-574 Lódz (PL); Mlynarski, Wojciech, 95-010 Anielin Swedowski (PL)
(74) Representative: Witek, Rafal

(56) References cited:
- LESZCZENKO PATRYCJA ET AL: "Towards Raman-Based Screening of Acute Lymphoblastic Leukemia-Type B (B-ALL) Subtypes", vol. 13, no. 21, 1 January 2021 (2021-01-01), pages 5483, XP093026243, Retrieved from the Internet <URL:https://www.ncbi.nlm.nih.gov/pmc/articles/PMC8582787/pdf/cancers-13-05483.pdf> DOI: 10.3390/cancers13215483
- TANDON SNEHA ET AL: "First report of t(5;11) KMT2A-MAML1 fusion in de novo infant acute lymphoblastic leukemia", CANCER GENETICS, ELSEVIER, AMSTERDAM, NL, vol. 248, 22 September 2020 (2020-09-22), pages 31 - 33, XP086337780, ISSN: 2210-7762, [retrieved on 20200922], DOI: 10.1016/J.CANCERGEN.2020.09.004
- PAUL G KEMPS ET AL: "B-cell lymphoblastic lymphoma with cutaneous involvement and a KMT2A gene rearrangement", AMERICAN JOURNAL OF HEMATOLOGY, NEW YORK, NY, US, vol. 95, no. 11, 15 April 2020 (2020-04-15), pages 1427 - 1429, XP071632690, ISSN: 0361-8609, DOI: 10.1002/AJH.25801

## Description

### FIELD OF THE INVENTION

The invention relates to a new method for diagnosing the presence of *KMT2A* gene rearrangement in acute lymphoblastic leukemia (ALL) cells in samples containing lymphoblasts isolated from the blood or bone marrow of patients suspected to suffer from leukemia using Raman spectroscopy. Identification of cells with *KMT2A* gene rearrangement is based on the result of measuring the integral intensity ratio of two bands: 1040 and 1008 cm⁻¹ (the Raman shift position may vary ± 3 cm⁻¹ bands due to the spectral resolution of the spectrometer used) observed on the Raman spectra of aldehyde fixed blood or bone marrow blasts.

### BACKGROUND OF THE INVENTION

Leukemias are a group of neoplastic diseases characterized by uncontrolled growth of precursor leukocytes with impaired physiological functions, inhibited in the process of differentiation. Leukemia cells primarily inhibit the myeloid niches, and in some cases also infiltrate non-myeloid organs, such as the lymph nodes, liver, or spleen. ALL, due to their high metabolic activity and intense proliferative processes, are effective in conventional chemotherapy, but the treatment process is long and burdening the patient. Leukemia relapses, which are the main cause of death in patients diagnosed with ALL, are an extremely important problem. The overall 5-year survival rate for acute lymphoblastic leukemia (ALL) in the paediatric population is almost 90%, while in adults it is only 60%. Relapses of leukemia, which are the main cause of death in patients diagnosed with ALL, are an extremely important problem [1]. According to statistics, in the United States alone, about 60,000 people suffer from leukemia annually, and about 24,000 die each year [2].

The most basic division of leukemias types includes acute and chronic, as well as lymphatic and myeloblastic. The type is distinguished by the type of cells that have undergone neoplastic transformation (lymphoid or myeloid lineage) or the course of the disease (acute and chronic leukemias). Leukemic cells (blasts) that are similar to each other in the histopathological picture may show different levels of chemosensitivity and have a different clinical picture. The source of this huge diversity lies in the extremely heterogeneous profile of disorders and abnormalities of the genomic leukemia cells that define the biological characteristics of cancer [3].

Immunophenotypic characteristics allows the distinction of ALL from B-cell progenitor acute lymphoblastic leukemia (BCP-ALL) and T-cell acute lymphoblastic leukemia (T-ALL) [4]. The diagnostic procedure for acute leukemia uses bone marrow cell aspirate, which undergoes morphological and cytochemical analysis, as well as immunophenotyping using flow cytometry and molecular and genetic tests. ALL is characterized by numerous genetic abnormalities that define its subtypes, which differ from each other in terms of cytomorphology, immunophenotype, chromosomal and gene aberrations, response to therapy and risk of recurrence.

As of now, nearly 20 molecular subtypes of BCP-ALL have been described, most of which have been defined by the presence of a particular fusion gene. Due to the fact that individual molecular subtypes of BCP-ALL may show a different response to chemotherapy, the detection of a specific genetic rearrangement in pathological lymphoblasts determines more intensive treatment, which in turn reduces the risk of leukemia recurrence [3,4].

*TEL-AML1* [t(12;21) (q13;q22)], *TCF3-PBX1* [t(1;19)(q23;p13)], BCR-ABL1 [*Philadelphia* chromosome (Ph) t(9;22)(q34;q11)-positive] fusion genes and rearrangement of the *lysine methyltransferase 2a* gene (*KMT2A-r*, formerly known as *MLL*), are the most recurrent disease-initiating genetic alterations in BCP-ALL. *TEL-AML1* accounts for 25% of childhood cases and is related to an excellent prognosis. Similarly, *TCF3-PBX1* subtype of ALL also shows a favourable outcome but increases the risk for ALL relapse in the central nervous system. In contrast, *Ph-positive* ALL, which occurs ten times more rarely in children as compared to adults, who represent 40-50%, is clearly associated with poor prognosis [7]. The unfavourable outcome of this subtype results from the constitutive activity of the *BCR-ABL1* oncoprotein. One of the relatively common subtypes of BCP-ALL, which is also characterized by an aggressive course combined with a poor prognosis, is ALL with the *KMT2A* rearrangement.

Increasing the frequency of ALL with *KMT2A-r* is reported in the infant population, where it is associated with high leucocytosis and frequent involvement of the central nervous system (CNS). At the cytogenetic level, balanced chromosomal translocations involving 11q23 are characteristic for BCP-ALL with rearrangements within the *KMT2A* (*KMT2A-r*) gene. They lead to functional disorders resulting in an abnormal process of growth and differentiation within the haematopoietic system and the immunophenotype of pro-B lymphoid cells. Moreover, *KMT2A-r* lymphoblasts are characterized by an unusually high level of resistance to treatment with L-asparaginase and glucocorticosteroids and increased chemosensitivity to cytarabine [5]. A particularly extremely poor treatment outcomes and noticeably short overall survival for *ALL-KMT2A-r* concerns the infant population, where survival rates are at the level of 30-40%.

The current standard protocols for the diagnosis of haematopoietic malignancies, including acute leukemias, are based on cytogenetic and molecular testing. Many diagnostic tools, such as immunophenotyping (flow cytometry), molecular techniques (RNA/DNA sequencing) or histochemical tests, require highly specialized equipment and multidisciplinary and experienced laboratory personnel, which is associated with significant costs. Moreover, in non-reference medical facilities, precise diagnosis of a specific molecular subtype of leukemia is extremely difficult or even impossible.

Although flow cytometry is undoubtedly a quick and sensitive method, its principle of operation is based on the use of a wide panel of antibodies, which makes it an extremely expensive technique (costs related to the purchase of antibodies - the more detailed the analysis, the higher the costs). Moreover, the lack of standardization and subjective interpretation of the data are significant limitations felt more acutely in difficult applications such as minimal residual disease (MRD) monitoring.

Classic karyotyping and fluorescence *in situ* hybridization (FISH) allow the identification of some subtypes of leukemia based on the assessment of structural disorders (deletions, duplications, translocations, or inversions) of significant size (>10Mbp) and numerical chromosome aberrations (hypodiploidy, hyperdiploidy). However, an undoubted and significant limitation resulting from the use of this methodology is the need to have active mitotic cells.

Document Leszczenko Patrycja et al: "Towards Raman-Based Screening of Acute Lymphoblastic Leukemia-Type B (B-ALL) Subtypes", vol. 13, no 21, (2021), p. 5483, discloses characterization and distinguishing cells isolated from the bone marrow of patients suffering from three subtypes of BCP-ALL, defined by gene rearrangements, i.e., BCR-ABL1, TEL-AML1 and TCF3-PBX1, using single-cell Raman imaging combined with multivariate statistical analysis. Document discloses that spectra collected from clinical samples were compared with single-cell spectra of B-cells collected from healthy donors, constituting the control group. It was demonstrated that Raman spectra of normal B cells strongly differ from spectra of their malignant counterparts, especially in the intensity of bands, which can be assigned to nucleic acids. Document discloses that the identification of leukemia subtypes could be automated with the use of chemometric methods and the clinical suitability of Raman imaging for the identification of spectroscopic markers characterizing leukemia cells.

Document Tandon Sneha et al: "First report of t(5;11) KMT2A-MAML1 fusion in de novo infant acute lymphoblastic leukemia" Cancer genetics, Elsevier, Amsterdam, NL, vol. 248, (2020), p. 31-33, discloses that a majority of infants with ALL harbor KMT2A gene rearrangement, approximately 94 different partner genes have been identified. The common rearrangements include t(4;11)(q21;q23)KMT2A-AFF1,t(11;19) (q23;p13.3)KMT2A-MLLT1 and t(9;11)(p22;q23)KMT2A-MLLT3. It was disclosed a novel translocation t(5;11)(q35;q23)KMT2A-MAML1 in newly diagnosed infant precursor B-ALL.

Document Paul G Kemps et al: "B-cell lymphoblastic lymphoma with cutaneous involvement and a KMT2A gene rearrangement", American journal of hematology, New york, NY, US, vol. 95, no. 11, 15 (2020), p. 1427-1429, discloses patient treatment with a B-cell lymphoblastic lymphoma (B-LBL) with typical cutaneous involvement and a KMT2A gene rearrangement that is common in (B-) ALL. 3 A KMT2A gene rearrangement was identified using fluorescence in situ hybridization (FISH). Targeted RNA-sequencing using the commercial Archer FusionPlex Lymphoma Kit revealed a KMT2A-MLLT1 (MLL-ENL) gene fusion and a WT1 frameshift mutation (supplementary methods). KMT2A FISH and targeted RNA-sequencing did not detect the KMT2A gene fusion in the bone marrow.

Considering the above, there is still demand for methods that would be enable fast, specific, simple, non-invasive, and low-cost diagnosis of ALL with *KMT2A-r.* The early diagnosis of this molecular subtype at the level of non-reference medical facilities is especially important.

These techniques are opposed by Raman spectroscopy (RS), which is a quick and nondestructive method providing biochemical characterization at the molecular level. It allows to obtain complete biochemical information without the need of use any tags and dyes. It was shown previously that primary BCP-ALL cells representing defined genetic subgroups can be discriminated from healthy cells based on the differences in the chemical composition using non-invasive and label-free Raman imaging [8]. The Raman spectroscopy-based approach of pathological cells analysis was previously reported, including a mixture with normal hematopoietic cells suspended in the solution [9,10] or B-ALL cell lines compared with normal B cells [11,12,14]. In work [13] it was reported that Raman spectroscopy was not only able to differentiate leukemic cells from normal lymphocytes, but it also enabled accurate classification of B-cell acute leukemia into the different differentiation/maturation stages [13]. It was also shown that the spectra of B-cell leukemia cell lines are characterized by a lower ratio of the intensity of the DNA/protein bands in relation to the healthy cells [14].

In a paper Leszczenko et al., [8], we attempt to characterize BCP-ALL cells being at the same stage of maturation (pre-B), depending on the presence of the specific genetic abnormalities, including *BCR-ABL1*, *TEL-AML1*, *TCF3-PBXI* gene fusions. The clinical usefulness of Raman imaging and spectroscopic markers for the characterization and identification of leukemic cells compared to normal B cells were shown. While we have achieved reproducible measurements that allowed us to distinguish between normal B cells and B-ALLs, several limitations remained. We anticipated that given the low degree of biochemical variation between studied BCP-ALL leukemia subtypes in terms of general protein and lipid composition, it would be difficult to discriminate them by means of unsupervised chemometric methods, such as e.g., PCA. Therefore, to identify reliable and robust spectral differences between ALL subtypes, a more advanced prediction model based on deep learning and a supervised approach is needed.

Unexpectedly, during further studied a method for specific identification of one of the ALL molecular subtype was obtained in this invention. Developed Raman-based method uses a simple chemometric approach of spectra analysis that allow to identify the presence of ALL blasts with *KMT2A-r.*

### SUMMARY OF THE INVENTION

The subject of the invention is a method of identifying the subtype of acute lymphoblastic leukemia with *KMT2A* gene rearrangement. Said method comprises the following steps:
(a) treating a sample containing lymphoblasts isolated from the blood or bone marrow of the diagnosed patient, with a solution of an aldehyde, preferably glutaraldehyde,
(b) recording the Raman spectra of the clinical samples from step (a);
(c) calculating the integral intensities ratio 1040/1008 cm⁻¹ ± 3 cm⁻¹,
wherein an increase value in this ratio, compared to the value obtained for a sample from a healthy patient is indicative of the identification of an subtype of ALL with *KMT2A-r.* Further advantageous embodiments according to the first aspect of the present invention have been specified in dependent claims 2 to 12.

In an advantageous embodiment, in step (a) the lymphoblasts are isolated from blood or bone marrow obtained from the patient.

In an advantageous embodiment, the Raman imaging of lymphoblasts is obtained from blasts isolated from peripheral blood if the number of blasts exceeds 90% of overall number of lymphocytes.

In an advantageous embodiment, in step (b) the samples for measuring Raman spectra are prepared as a suspension of cells in physiological saline or PBS.

In an advantageous embodiment, the Raman spectra of blasts from step (b), are recorded using Raman spectrometer with excitation of a 532 nm laser equipped with a water immersive objective.

In an advantageous embodiment, in step (b) single Raman spectra are recorded from at least 2 different points of at least 20 single measured cells.

In an advantageous embodiment, the Raman spectra of the lymphoblasts measured at stage (b) are recorded in the fingerprint range (500-1800 cm⁻¹).

In an advantageous embodiment, in step (b) Phe bands are observed at 1040 and 1008 cm⁻¹ ± 3 cm⁻¹, wherein for the presence of *KMT2A* gene rearrangement in cells fixed with aldehyde solution, the band intensity increases at 1040 cm⁻¹ and the band ratio of 1040/1008 cm⁻¹ increases.

In an advantageous embodiment, in step (b) for cells fixed with an aldehyde solution with the presence of *KMT2A*-r, the calculated average value of 1040/1008 cm⁻¹ ratio ± 3 cm⁻¹ at stage (c) is equal or higher than 0.95.

In an advantageous embodiment, at step (a) it relates to biological material, which are blood or bone marrow collected from patients at the time of ALL diagnosis.

In an advantageous embodiment, at step (a) it relates to lymphoblasts isolated from blood or bone marrow.

In an advantageous embodiment, the method relates to identifying ALL cells with the presence of a rearrangement within the *KMT2A* gene.

### DETAILED DESCRIPTION OF THE INVENTION

In an exemplary implementation, there is disclosed the method of identifying the subtype of acute lymphoblastic leukemia with *KMT2A* gene rearrangement comprising:
(a) treating a blood or marrow sample containing leukemic cells (lymphoblasts) with a 0.2 µm filtered 0.5% aldehyde fixative;
(b) flushing the cells three times for 5 minutes with saline or PBS (phosphate buffer saline)
(c) collecting Raman spectra of the single cells from suspension in saline / PBS;
(d) calculating the Raman integral intensities ratio of 1040 cm⁻¹ to 1008 cm⁻¹ bands present in the Raman spectra of lymphoblasts collected from patients.
(e) it is confirmed that the value of 1040/1008 cm⁻¹ higher than 0.95 for the Raman bands is indicative of the presence of *KMT2A-r* in ALL gene.

In an advantageous embodiment, in stages (a) and (b) the method relates to samples of biological material that are cells isolated from the blood (when lymphoblasts are present in the peripheral blood) or the bone marrow of the patients.

In an advantageous embodiment, isolation of peripheral blood lymphoblasts can be used when the observed blastosis is higher than 90%.

In an advantageous embodiment, in stage (c) the measurement of the intensity ratio of the 1040/1008 cm⁻¹ bands is performed by analysing the Raman signal from spectra obtained using a confocal Raman imaging system, and the measurement of Raman imaging was performed using a water immersion objective. It is advantageous to use a Raman spectrometer with an imaging function as it allows the measurement of multiple spectra from a single cell, thus tracking and identifying variability within a sample by deriving a standard deviation value.

In an advantageous embodiment, in stage (c) the cell imaging measurement is conducted with preparations placed on a medium that does not exhibit a Raman signal in the spectral range of 900-1100 cm⁻¹, such as, for example, CaF₂, BaF₂ or ZnSe.

In an advantageous embodiment, in stage (c) the spectra are obtained by excitation of the samples using a laser with a wavelength of 532 nm and a laser power in the range of 20-30 mW.

Preferably, the measurement of the 1040/1008 cm⁻¹ integral intensity ratio that assessing the presence of the *KMT2A* fusion gene is performed in leukemic cells (blasts) obtained from the blood or bone marrow of patients during the routine diagnosis of the acute lymphoblastic leukemia subtype.

In an advantageous embodiment, in stage (a) the leukemic cell samples have been fixed for 10 minutes with 0.5% glutaraldehyde (GA) solution. It was confirmed that lower and higher GA concentrations is important for identification of BCP-ALL cells with *KMT2A-r* cells based on results from 1040/1008 cm⁻¹ intensities ratio. During the analysis of cells fixed with 0.1, 0.5 and 2.5% GA it was noticed that proposed method requires to work on cells fixed with 0.5% GA.

In an advantageous embodiment, the characteristic bands in the Raman spectra are located within the following ranges: from 980 to 1022 cm⁻¹ and from 1022 to 1055 cm⁻¹.

In an advantageous embodiment, in stage (d) it is accepted that the 1040/1008 cm⁻¹ ratio for the patients suffering from another BCP-ALL subtype are lower than 0.95.

In an advantageous embodiment, in stage (d) it is accepted that the 1040/1008 cm⁻¹ ratio for the healthy samples (leukocytes) are lower than 0.9.

In an advantageous embodiment, in stage (d) it is accepted that the 1040/1008 cm⁻¹ ratio for the patients suffering from BCP-ALL with *KMT2A-r* subtype are equal or higher than 0.95.

When the 1040/1008 cm⁻¹ band intensity ratio is greater than 0.95, the presence of the *KMT2A* fusion gene is detected. The above-mentioned numerical values of the position of the bands may change due to the individual variability and the spectral resolution of the spectrometer by approx. 3-5 cm⁻¹.

The advantage of the described method is that it can be used to assess the presence of rearrangements within the *KMT2A* gene in samples, and the analysis is based on a rapid and marker-free evaluation of the recorded signal. Another advantage of the invention is the possibility to automate the protocol and conduct diagnostics by persons trained to operate the apparatus without the need for the presence of a specialist in the field of molecular spectroscopy.

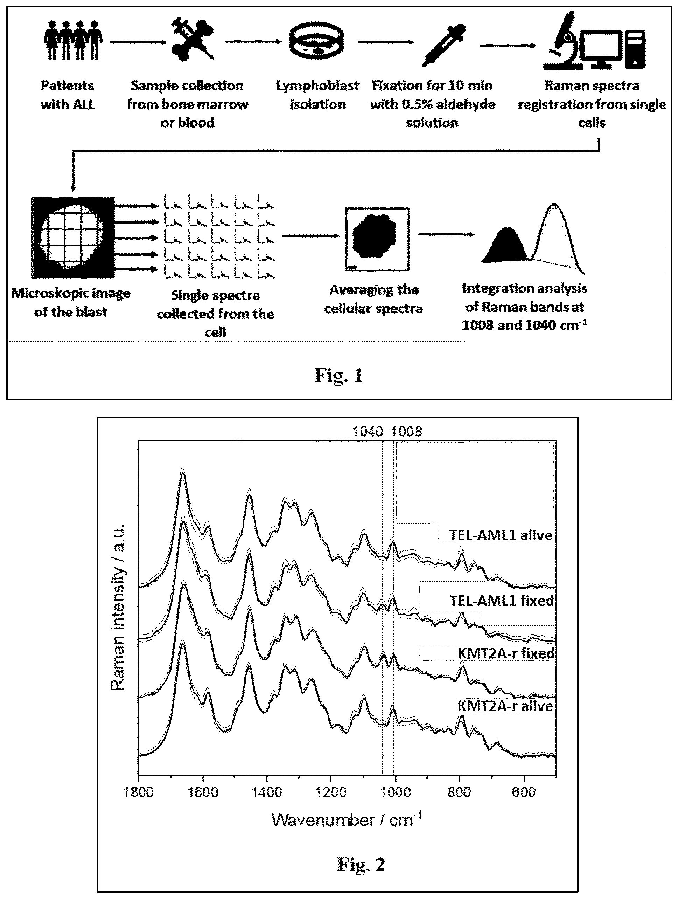

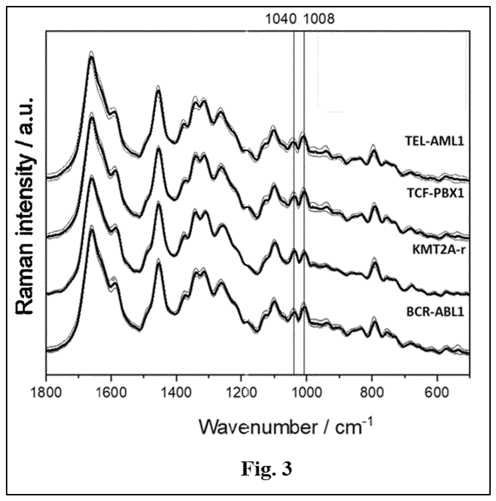

Fig. 1 shows a scheme of the workflow of preparation, measurements, analysis; Fig. 2 illustrates a comparison of mean spectra of leukemic blasts with *KMT2A* and *TEL-AML1* rearrangements before (live cells) and after fixation with 0.5% glutaraldehyde along with an indication of the 1040 and 1008 cm⁻¹ bands; Fig. 3 shows a comparison of mean spectra of leukemic blasts with the fusion genes: (a) *KMT2A-r*, (b) *BCRABL1*, (c) *TCF3-PBX1,* (d) *TEL-AML1*;

### DESCRIPTION OF THE DRAWINGS

Fig. 1 Schematic representation of the workflow of preparation, measurements, analysis.
Fig. 2 Comparison of averaged Raman spectra of living (unfixed) leukemia blasts with *KMT2A-r* and *TEL-AML1* mutation and fixed with 0.5% glutaraldehyde along with an indication of the 1040 and 1008 cm⁻¹ bands. The figure illustrates impact of 0.5% GA fixation on the Raman profile of BCP-ALL cells with *KMT2A-r* and *TEL-AML1* mutations.
Figure 3 illustrates a comparison of mean Raman spectra of leukemia blasts with the fusion genes: *BCR-ABL1*, *TCF3-PBX1*, *TEL-AML1 ,* and *KMT2A-r.* Mean Raman spectra of the whole cells fixed with 0.5% GA with the standard deviation (shadow), measured using a 532 nm laser. Spectra presented in the fingerprint range of (1800 -500 cm⁻¹).

### DETAILED DESCRIPTION OF THE EMBODIMENTS

Example 1. The invention is illustrated in the following embodiment, which does not limit the protection of the invention as sought.

### Treating samples containing isolated lymphoblasts

The general procedure is shown in Fig. 1. Samples containing lymphoblasts isolated from bone marrow or peripheral blood of patients with suspected B-cell acute lymphoblastic leukemia (BCP-ALL) on the day of BCP-ALL diagnosis were stored at -156 °C in liquid nitrogen gas phase. For the study samples with four typical BCP-ALL gene rearrangements were selected, i.e., *BCR-ABL1*, *TEL-AML1*, *TCF3-PBX1* and *KMT2A-r.* Prior to Raman imaging measurements, BCP-ALL samples were removed from the gaseous liquid nitrogen phase and thawed rapidly in a water bath (37 °C). Following a flushing step with PBS (3×) at room temperature (300 ×g, 5 minutes), the viability of the leukemic samples was assessed using the trypan blue exclusion method.

### Fixation of the samples

Lymphoblasts were then fixed with 0.5% glutaraldehyde (GA; Sigma-Aldrich) for 10 minutes at room temperature (Fig. 1) and then washed in suspension three times with physiological saline buffer or PBS to remove any excess of the fixer. In the last step, cells after centrifugation were resuspended in physiological saline buffer (about 500 µl) and stored in Eppendorf tubes (Eppendorf Conical Tubes) at 4 °C until Raman measurements. Immediately before measurements, cells were centrifuged (300 ×g, 5 minutes) to obtain a pellet of cells, from which approximately 200 µL was aspirated and placed directly on a calcium fluoride (CaF₂) slide.

### Raman measurements

Raman imaging measurements were performed using a WITec Alpha 300 confocal Raman microscope (Ulm, Germany) equipped with a CCD detector (Andor Technology Ltd) and 600 grooves/mm grating. For single cell measurements, a 63x water immersion objective (Zeiss W Plan-Apochromat 63x, NA=1) was used, which was then immersed in a cell suspension placed on a CaF₂ medium (25x2 mm, Crystran Ltd). In case of observing too high concentration of cells under the microscope, the suspension was diluted by adding PBS in the volume of approx. 200-300 µL. Cell morphology was assessed based on the microscopic image for each sample to measure only round cells with no visible signs of abnormalities. Each tested sample was measured as several independent biological replicates (samples from minimum 5 different patients (n=5)). About 50 cells per sample were measured for each set of cells fixed with 0.5% glutaraldehyde. Raman spectra were collected using an excitation laser with a wavelength of 532 nm. Raman images were collected with 1 µm step with an integration time of 0.5 s per spectrum and laser power of ~20 mW (beam power measured before the objective).

### Spectral data pre-processing

The basic procedure for pre-processing of Raman data involved baseline correction and cosmic ray removal. For this purpose, functions available in the WITec Project Plus 5.1 software (Ulm, Germany) were used. In the first stage of the analysis of Raman images, the cosmic ray signals recorded by the CCD camera were removed (Cosmic Ray Removal, filter size - 3, dynamic factor - 8) and the baseline correction was applied using the polynomial function (degree 3). Then, using the same software for each recorded Raman image, k-means cluster analysis (KMCA) was performed using the Manhattan distance calculation and the Ward clustering algorithm to extract one average spectrum of the whole cell and separate the cellular spectra from the background signal (Fig. 1).

### Spectral data analysis

When compared Raman spectra of aldehyde fixed cells to the spectra of live cells (Fig. 2), one can observe reduced intensity of the Raman bands characteristic for hemoproteins (750, 1130, 1315, and 1585 cm⁻¹) and increasing the intensity of the band at approx. 1040 cm⁻¹. The decrease in the intensity of the bands characteristic for the cytochrome (one of the hemoproteins) is the result of its oxidation by aldehyde during the fixation process. In turn, the appearance of the 1040 cm⁻¹ band present in the spectra of fixed cells is the result of protein cross-linking as a result of a reaction with aldehyde function group. The higher intensity of the band at 1040 cm⁻¹ observed for fixed ALL cells with *KMT2A-r* may be related to changes in the Phe conformation in the proteins of these cells. Variations in the intensity ratios of the intensity of the bands 1040 and 1008 cm⁻¹ are also seen in the averaged spectra (Fig. 3).

### Calculation of the integral intensity ratio of 1040/1008 cm⁻¹

The integral intensity ratio of 1040 to 1008 cm⁻¹ Raman bands was calculated for the average spectra of each measured cell. To verify the statistical significance of the observed differences in the calculated values of the 1040/1008 cm⁻¹ ratio for all 4 ALL subtypes, analysis of variance using the ANOVA method with Tukey's test at the significance level equal to 0.05 was carried out (calculation done in the OriginPro 2020 software).

### Results

As a result of the conducted experiments and data analysis, the obtained result - illustrated in Fig. 3 - indicating that the value of the quantitative ratio of the 1040/1008 cm⁻¹ bands **is significantly higher in the samples with the presence of *KMT2A-r*** when compared to the spectra obtained from samples collected from patients with other molecular subtypes of BCP-ALL leukemias.

In Example 1, the ratio of the average integral intensity ratio of Raman bands at 1040 to 1008 cm⁻¹ calculated from Raman spectra of samples from patients diagnosed for BCP-ALL with a rearrangement within the *KMT2A* gene is greater than 0.95 when cells are fixed with 0.5% GA.

### Annotation

The experiments were conducted with the consent of the Bioethics Committee of the Medical University of Lodz no. RNN/270/19/KE (extension KE/30/21) of May 14, 2019. Blood from volunteers and bone marrow from patients were collected after obtaining informed consent.

### Reference literature

1. Konturek, S.; Brzozowski, T. *Fizjologia czlowieka. Tom 1. Fizjologia ogólna, krew i mi* 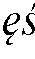 śnie; W 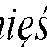 gierska, D., Ed.; Wydanie VI.; Wydawnictwo Uniwersytetu Jagiellońskiego: Kraków, 2003; ISBN 83-233-1628-7.
2. https://seer.cancer.gov/statfacts/html/leuks.html Available online: https://seer.cancer.gov/statfacts/html/leuks.html.
3. Baiocchi, R.A. Classification of malignant lymphoid disorders. In Williams Hematology; McGraw-Hill Education, 2016; pp. 1493-1503 ISBN 978-0-07-183301-1.
4. Larson, R.A. Acute lymphoblastic leukemia. In Williams Hematology; McGraw-Hill Education, 2016; pp. 1505-1526 ISBN 978-0-07-183301-1.
5. Pastorczak, A.; Domka, K.; Fidyt, K.; Poprzeczko, M.; Firczuk, M. Mechanisms of immune evasion in acute lymphoblastic leukemia. Cancers (Basel). 2021, 13, 1-25, doi: 10. 3390/cancers 13071536.
6. Winters, A.C.; Bernt, K.M. MLL-rearranged leukemias- An update on science and clinical approaches. Front. Pediatr. 2017, 5, 11-13, doi:10.3389/fped.2017.00004.
7. Roberts K.G., Mullighan C.G. The biology of B-progenitor acute lymphoblastic leukemia. Cold Spring Harb. Perspect. Med. 2020;10:1-22, doi: 10.1101/cshperspect.a034835.
8. Leszczenko P, Borek-Dorosz A, Nowakowska AM, Adamczyk A, Kashyrskaya S, Jakubowska J, Z 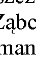 bczyńska M, Pastorczak A, Ostrowska K, Baranska M, Marzec KM, Majzner K. Towards Raman-Based Screening of Acute Lymphoblastic Leukemia-Type B (B-ALL) Subtypes. Cancers (Basel). 2021 Oct 31;13(21):5483. doi: 10.3390/cancers13215483.
9. Bai Y., Yu Z., Yi S., Yan Y., Huang Z., Qiu L. Raman spectroscopy-based biomarker screening by studying the fingerprint characteristics of chronic lymphocytic leukemia and diffuse large B-cell lymphoma. J. Pharm. Biomed. Anal. 2020;190:113514. doi: 10.1016/jjpba.2020.113514.
10. Féré M., Gobinet C., Liu L.H., Beljebbar A., Untereiner V., Gheldof D., Chollat M., Klossa J., Chatelain B., Piot O. Implementation of a classification strategy of Raman data collected in different clinical conditions: Application to the diagnosis of chronic lymphocytic leukemia. Anal. Bioanal. Chem. 2020;412:949-962. doi: 10.1007/s00216-019-02321-z
11. Hassoun M., Köse N., Kiselev R., Kirchberger-Tolstik T., Schie I.W., Krafft C., Popp J. Quantitation of acute monocytic leukemia cells spiked in control monocytes using surface-enhanced Raman spectroscopy. Anal. Methods. 2018;10:2785-2791. doi: 10.1039/C8AY01046C.
12. Managò S., Valente C., Mirabelli P., De Luca A.C. Discrimination and classification of acute lymphoblastic leukemia cells by Raman spectroscopy. Opt. Sensors. 2015;9506:95060Z. doi: 10.1117/12.2179486.
13. Managò S., Zito G., De Luca A.C. Raman microscopy based sensing of leukemia cells: A review. Opt. Laser Technol. 2018;108:7-16. doi: 10.1016/j.optlastec.2018.06.034
14. Managò S., Valente C., Mirabelli P., Circolo D., Basile F., Corda D., De Luca A.C. A reliable Raman-spectroscopy-based approach for diagnosis, classification and follow-up of B-cell acute lymphoblastic leukemia. Sci. Rep. 2016;6:1-13. doi: 10.1038/srep24821.

## Claims

1. A method of identifying the subtype of acute lymphoblastic leukemia with *KMT2A* gene rearrangement, **characterised in that** said method comprises the following steps:
(a) treating a sample containing lymphoblasts isolated from the blood or bone marrow of the diagnosed patient with a solution of an aldehyde, preferably glutaraldehyde,
(b) recording the Raman spectra of the clinical samples from step (a);
(c) calculating the integral intensities ratio 1040/1008 cm⁻¹ ± 3 cm⁻¹,
wherein an increase value in this ratio, compared to the value obtained for a sample from a healthy patient is indicative of the identification of an subtype of ALL with *KMT2A-r.*

2. The method according to claim 1, wherein in step (a) the lymphoblasts are isolated from blood or bone marrow obtained from the patient.

3. The method according to claim 1, wherein the Raman imaging of lymphoblasts is obtained from blasts isolated from peripheral blood if the number of blasts exceeds 90% of overall number of lymphocytes.

4. The method according to claim 1, wherein in step (b) the samples for measuring Raman spectra are prepared as a suspension of cells in physiological saline or PBS.

5. The method according claim 1, wherein the Raman spectra of blasts from step (b), are recorded using Raman spectrometer with excitation of a 532 nm laser equipped with a water immersive objective.

6. The method according to claims 1, wherein in step (b) single Raman spectra are recorded from at least 2 different points of at least 20 single measured cells.

7. The method according claims 1, wherein the Raman spectra of the lymphoblasts measured at stage (b) are recorded in the fingerprint range (500-1800 cm⁻¹).

8. The method according to claims 1, wherein in step (b) Phe bands are observed at 1040 and 1008 cm⁻¹ ± 3 cm⁻¹, wherein for the presence of *KMT2A* gene rearrangement in cells fixed with aldehyde solution, the band intensity increases at 1040 cm⁻¹ and the band ratio of 1040/1008 cm⁻¹ increases.

9. The method according to claims 1, 6 and 8, wherein in step (b) for cells fixed with an aldehyde solution with the presence of *KMT2A-r,* the calculated average value of 1040/1008 cm⁻¹ ratio ± 3 cm⁻¹ at stage (c) is equal or higher than 0.95.

10. The method according to claim 1, wherein at step (a) it relates to biological material, which are blood or bone marrow collected from patients at the time of ALL diagnosis.

11. The method according to claims 1 to 10, wherein at step (a) it relates to lymphoblasts isolated from blood or bone marrow.

12. The method according to claims 1 to 10, wherein it relates to identifying ALL cells with the presence of a rearrangement within the *KMT2A* gene.

## Patentansprüche

1. Methode zur Identifizierung des Subtyps der akuten lymphatischen Leukämie mit *KMT2A*-Genneuanordnung, **dadurch gekennzeichnet, dass** die Methode folgende Schritte umfasst:
(a) Behandeln einer Probe, die Lymphoblasten enthält, die aus dem Blut oder Knochenmark des diagnostizierten Patienten isoliert wurden, mit einer Lösung eines Aldehyds, vorzugsweise Glutaraldehyd,
(b) Aufzeichnen der Raman-Spektren der klinischen Proben aus Schritt (a);
(c) Berechnen des integralen Intensitätsverhältnisses 1040/1008 cm⁻¹ ± 3 cm⁻¹,
wobei ein Anstiegswert in diesem Verhältnis im Vergleich zu dem Wert, der für eine Probe von einem gesunden Patienten erhalten wurde, auf die Identifizierung eines Subtyps von ALL mit *KMT2A-r* hinweist.

2. Methode nach Anspruch 1, wobei in Schritt (a) die Lymphoblasten aus dem vom Patienten gewonnenen Blut oder Knochenmark isoliert werden.

3. Methode nach Anspruch 1, wobei die Raman-Bildgebung von Lymphoblasten aus Blasten gewonnen wird, die aus peripherem Blut isoliert sind, wenn die Anzahl der Blasten 90 % der Gesamtzahl der Lymphozyten überschreitet.

4. Methode nach Anspruch 1, wobei in Schritt (b) die Proben zum Messen von Raman-Spektren als Suspension von Zellen in physiologischer Kochsalzlösung oder PBS vorbereitet werden.

5. Methode nach Anspruch 1, wobei die Raman-Spektren von Blasten aus Schritt (b) unter Verwendung eines Raman-Spektrometers mit Anregung eines 532-nm-Lasers, der mit einem Wassereintauchobjektiv ausgestattet ist, aufgezeichnet werden.

6. Methode nach Anspruch 1, wobei in Schritt (b) einzelne Raman-Spektren von mindestens 2 verschiedenen Punkten von mindestens 20 einzeln gemessenen Zellen aufgezeichnet werden.

7. Methode nach Anspruch 1, wobei die Raman-Spektren der im Stadium (b) gemessenen Lymphoblasten im Fingerabdruckbereich (500-1800 cm⁻¹) aufgezeichnet werden.

8. Methode nach Anspruch 1, wobei in Schritt (b) Phe-Bänder bei 1040 und 1008 cm⁻¹ ± 3 cm⁻¹ beobachtet werden, wobei für das Vorhandensein einer *KMT2A-*Genneuanordnung in mit Aldehydlösung fixierten Zellen die Bandintensität bei 1040 cm-¹ zunimmt und das Bandverhältnis von 1040/1008 cm⁻¹ zunimmt.

9. Methode nach Anspruch 1, 6 und 8, wobei in Schritt (b) für mit einer Aldehydlösung fixierte Zellen mit dem Vorhandensein von *KMT2A-r* der berechnete Durchschnittswert von 1040/1008 cm⁻¹ ± 3 cm⁻¹ in Schritt (c) gleich oder höher als 0,95 ist.

10. Methode nach Anspruch 1, wobei es sich bei Schritt (a) auf biologisches Material bezieht, bei dem es sich um Blut oder Knochenmark handelt, das zum Zeitpunkt der ALL-Diagnose von Patienten entnommen wurde.

11. Methode nach einem der Ansprüche 1 bis 10, wobei es sich bei Schritt (a) auf Lymphoblasten bezieht, die aus Blut oder Knochenmark isoliert sind.

12. Methode nach einem der Ansprüche 1 bis 10, wobei es sich um die Identifizierung ALL-Zellen mit dem Vorhandensein einer Neuanordnung innerhalb des KMT2A-Gens handelt.

## Revendications

1. Méthode d'identification du sous-type de leucémie lymphoblastique aiguë avec réarrangement du gène *KMT2A,* **caractérisée en ce que** ladite méthode comprend les étapes suivantes :
(a) le traitement d'un échantillon contenant des lymphoblastes isolés du sang ou de la moelle osseuse du patient diagnostiqué avec une solution d'un aldéhyde, de préférence de glutaraldéhyde,
(b) l'enregistrement des spectres Raman des échantillons cliniques de l'étape (a) ;
(c) le calcul du rapport d'intensités intégrales 1040/100 cm⁻¹ ± 3 cm⁻¹,
dans laquelle une valeur d'augmentation de ce rapport, par rapport à la valeur obtenue pour un échantillon d'un patient sain, est indicative de l'identification d'un sous-type de LLA avec *KMT2A-r*

2. Méthode selon la revendication 1, dans laquelle à l'étape (a), les lymphoblastes sont isolés du sang ou de la moelle osseuse obtenus auprès du patient.

3. Méthode selon la revendication 1, dans laquelle l'imagerie Raman de lymphoblastes est obtenue à partir de blastes isolés du sang périphérique si le nombre de blastes dépasse 90 % du nombre total de lymphocytes.

4. Méthode selon la revendication 1, dans laquelle à l'étape (b), les échantillons pour mesurer les spectres Raman sont préparés sous forme de suspension de cellules dans du sérum physiologique ou du PBS.

5. Méthode selon la revendication 1, dans laquelle les spectres Raman des blastes de l'étape (b), sont enregistrés à l'aide d'un spectromètre Raman avec excitation d'un laser de 532 nm équipé d'un objectif immersif dans l'eau.

6. Méthode selon la revendication 1, dans laquelle à l'étape (b), des spectres Raman simples sont enregistrés à partir d'au moins 2 points différents d'au moins 20 cellules mesurées simples.

7. Méthode selon la revendication 1, dans laquelle les spectres Raman des lymphoblastes mesurés à l'étape (b) sont enregistrés dans la plage d'empreintes digitales (500-1800 cm⁻¹).

8. Méthode selon la revendication 1, dans laquelle à l'étape (b), des bandes Phe sont observées à 1040 et 1008 cm⁻¹ ± 3 cm⁻¹, dans laquelle pour la présence d'un réarrangement du gène *KMT2A* dans des cellules fixées avec une solution d'aldéhyde, l'intensité de bande augmente à 1040 cm⁻¹ et le rapport de bande de 1040/1008 cm⁻¹ augmente.

9. Méthode selon l'une de la revendication 1, la revendication 6 et la revendication 8, dans laquelle à l'étape (b) pour les cellules fixées avec une solution d'aldéhyde en présence de *KMT2A-r,* la valeur moyenne calculée de 1040/1008 cm⁻¹ ± 3 cm⁻¹ à l'étape (c) est égale ou supérieure à 0,95.

10. Méthode selon la revendication 1, dans laquelle à l'étape (a), elle concerne un matériau biologique, qui est du sang ou de la moelle osseuse, prélevé chez des patients au moment du diagnostic de LLA.

11. Méthode selon les revendications 1 à 10, dans laquelle à l'étape (a), elle concerne des lymphoblastes isolés du sang ou de la moelle osseuse.

12. Méthode selon les revendications 1 à 10, dans laquelle elle concerne l'identification des cellules LLA avec la présence d'un réarrangement au sein du gène *KMT2A.*
